# EUROPEAN PATENT APPLICATION

(11) **EP 3 395 334 A1**
(43) Date of publication of application: **31.10.2018**
(21) Application number: 16877579.9
(22) Date of filing: 06.12.2016
(51) Int. Cl.: A61K 9/52, A61K 31/4035, A61K 47/38, A61K 47/34, A61K 47/32, A61P 17/06

(54) **APREMILAST SUSTAINED RELEASE PREPARATION**

(30) Priority: 24.12.2015 CN 201510982528
(71) Applicant: Jiangsu Hengrui Medicine Co. Ltd., Jiangsu 222047 (CN)
(72) Inventor: PAN, Kai, Lianyungang Jiangsu 222047 (CN); LIU, Kai, Lianyungang Jiangsu 222047 (CN); GUO, Chenning, Lianyungang Jiangsu 222047 (CN); LIU, Tong, Lianyungang Jiangsu 222047 (CN)
(74) Representative: Regimbeau
(86) International application number: PCT/CN2016/108709
(87) International publication number: WO 2017/107768

(57) **Abstract**

The present invention relates to an apremilast sustained release preparation. Specifically, the present invention relates to an apremilast composition which is a sustained release preparation having a high bioavailability in the body. The present invention provides a sustained release drug preparation containing an apremilast sustained release component and a site-specific release component therefor. Within in vitro release testing, the release speed is steady and constant.

## Description

### FIELD OF THE INVENTION

The present invention relates to a sustained release formulation of poorly soluble drug apremilast. Specifically, the present invention relates to a sustained release formulation comprising a sustained release component and a site-specific release component. The formulation has a gentle release during the *in vitro* release test, which can ensure that the plasma concentration is gentle and the effect is long-lasting.

### BACKGROUND OF THE INVENTION

Apremilast, chemical name of N-[2-[(1S)-1(3-ethoxy-4-methoxyphenyl)-2-(methylsulfonyl)ethyl]-2,3-2*H*-1,3-dioxy-1 *H*-isoindol-4-yl]acetamide, has the structure as shown below:

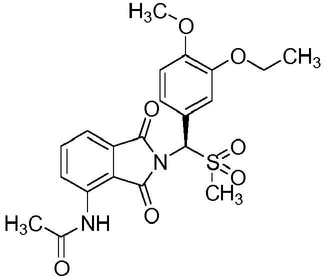

Apremilast is a small-molecule phosphodiesterase inhibitor specifically targeting cyclic adenosine monophosphate (cAMP). Inhibition of PDE4 results in an increase in intracellular cAMP levels, and the inflammatory response is down-regulated by modulating the expression of TNF-α, IL-23, and other inflammatory cytokines.

Apremilast is a poorly soluble compound, a BCSIV drug, virtually insoluble in water and can be used to treat psoriasis. Psoriasis is a common skin disease characterized by chronic inflammatory lesion that troubles patients for a long time and is not easily to be treated. At present, there are many clinical applications of biological agents. The main biological agents include TNF-α inhibitors, interleukin-12, 23 (IL-12/IL-23) inhibitors, and antibodies targeting B cells and T cells. The biological agents currently used in the treatment of psoriasis have rapidly developed and have significant clinical effect. However, the biological agents are generally administered by injection, and psoriasis requires long-term treatment control. Therefore, during the systemic and long-term treatment of psoriasis, there are problems such as low compliance and easy tolerance for the biological agents. In view of the above situation, apremilast, as a medicament which can be administrated orally in the treatment of psoriasis, has a great advantage.

The apremilast formulations that have been on the market at present are ordinary tablets. For long-term use, the tablets are administrated twice daily, 30 mg for once, with an interval of 12 hours. The apremilast sustained-release tablets can change the frequency to once a day, so that the patients' compliance is increased, and the fluctuation of plasma concentration is reduced, which can greatly reduce the side effects during medication. The sustained-release tablets therefore have a great advantage for patients with long-term medication.

Because the gastrointestinal side effect of apremilast is serious, and the frequency of side effect is closely related to the plasma concentration, the dosage of the currently available immediate release tablets need to be increased day by day via titration. The specific way of administration is shown in the table below, which results in low compliance of patients.

| Day 1 | Day 2 | | Day 3 | | Day 4 | | Day 5 | | Day 6 and later | |
|---|---|---|---|---|---|---|---|---|---|---|
| a.m. | a.m. | p.m. | a.m. | p.m. | a.m. | p.m. | a.m. | p.m. | a.m. | p.m. |
| 10 mg | 10 mg | 10 mg | 10 mg | 20 mg | 20 mg | 20 mg | 20 mg | 30 mg | 30 mg | 30 mg |

Based on the above reasons, there is a pressing need in clinical practice for a sustained release formulation of apremilast that has a more gentle plasma concentration in the body after administration. Specifically, such an apremilast sustained release formulation can reduce the frequency of daily medications and increase the patients' compliance, which is necessary for patients who require long-term medication. At the same time, such an apremilast sustained release formulation has a gentle release of drug after administration and can be slowly released in the body, which reduces the fluctuation of plasma concentration produced by twice daily administration and can greatly reduce the incidence of side effect.

WO2013119607 discloses a sustained release pharmaceutical composition comprising a high molecular polymer such as hydroxypropyl cellulose as a sustained release matrix material. As shown by the drug release data in its specification, the release rate of the sustained release drug exhibits a linear relationship and cannot achieve a good sustained release effect.

US20140370092 discloses an oral formulation prepared from different sustained release components, one of which is a sustained release component coated with a single-layer coating agent and the other of which is a sustained release component coated with a double-layer coating agent. The releasing rate of the tablets or capsules prepared according to the method is not gentle enough, and the release amount is not constant. The technical solution cannot solve the existing problems well, that is, reducing the frequence of medications, ensuring low fluctuation of plasma concentration in patients, increasing drug bioavailability and reducing the side effects during medication.

### SUMMARY OF THE INVENTION

The present invention relates to a sustained release formulation comprising a sustained release component of apremilast and a site-specific release component of apremilast. By combining two components with different drug release behaviors, the release behavior of apremilast after oral administration is regulated, so as to ensure that the plasma concentration of apremilast after oral administration is more constant than that of an ordinary tablet, the fluctuation of plasma concentration is reduced, the incidence of side effect is reduced significantly, and the duration of action is longer, thereby reducing the frequency of medications.

Specifically, a sustained release component I of apremilast and a site-specific release component II of apremilast are mixted in a certain weight ratio in the present invention. The site-specific release component II is prepared from apremilast or a pharmacologically acceptable salt or solvate thereof and a site-specific release coating. The sustained release component I of apremilast is prepared from apremilast or a pharmacologically acceptable salt or solvate thereof and a sustained release material, which is a matrix-type sustained release component. The active ingredient apremilast or a pharmacologically acceptable salt or solvate thereof is present in an amount of 5-20% by weight, relative to the weight of the corresponding sustained release component I or the site-specific release component II.

In order to achieve a more preferable sustained release effect, the site-specific release component II can also contain a sustained release material, so that the site-specific release component can release the drug slowly after reaching at a specific area in the body, which prolongs the administration interval, is beneficial to the absorption of the drug in the body, and can reduce the drug side effects. In order to achieve the optimal sustained release effect of the pharmaceutical formulation, the researchers conducted detailed studies on the combination ratio of the sustained release component I and the site-specific release component II in the pharmaceutical formulation, and found that when the weight ratio of the sustained release component I to the site-specific release component II is 1:8-8:1, the sustained release rate of the pharmaceutical formulation is good, and when the weight ratio is 1:6-6:1, the sustained release rate is better, and more preferably 1:5-4:1, specifically 2/5, 3/5, 4/5, 1/4, 1/3, 2/3, 3/4, 1/2, 1/1, 2/1, 3/2, or 3/1.

The pharmaceutical formulation according to the present invention comprises at least one site-specific release coating which can be a gastric coating or an enteric coating. Any enteric coating can be used in the present invention, including but not limited to, one or more of ethyl cellulose, hydroxypropyl cellulose, hydroxypropyl methyl cellulose, hydroxypropyl methyl cellulose titanate (HMPCP), polyvinyl acetate phthalate (PVAP), sodium alginate, cellulose acetate phthalate (CAP), shellac, terpolymer of methyl methacrylate, methacrylic acid and butyl acrylate, a solution or dispersion of copolymer of methyl acrylic acid and methacrylate, acetyltitanium cellulose, hydroxypropyl methylcellulose phthalate, hydroxypropyl methylcellulose acetate succinate, poly(vinyl acetate phthalate), acrylic resin (trade name: Eudragit), under the specific trade names of USNF A (Eudragit L™), B (Eudragit S™), C (Eudragit L 100-55™), Eudragit NE 30D, Eudragit E, Eudragit RL, Eudragit RS, cellulose acetate trimellitate, and lac.

In addition, the enteric coating used in the formulation of the present invention can be a single layer or multiple layers. The thickness of the coating can be readily determined by the person skilled in the art, but it must be sufficient to protect the formulation in an acidic stomach environment. The weight of the enteric coating is 1-40%, more preferably 2-30%, and most preferably 2-20% based on the total weight of the site-specific release component II, and specifically 6%, 7%, 8%, 9%, 10%, 11%, 12%, 13%, 14%, 15%, 16%, 17%, 18%, or 19%.

The sustained release material (also called as sustained release matrix material) used in the sustained release component I and the site-specific release component II of the present invention is one or more of polyoxyethylene, hypromellose, polyvinyl acetate and polyvinylpyrrolidone polymer (Kollidon SR), hydroxypropyl cellulose, copolymer of polyalkyl saccharose or polyalkyl pentaerythritol and acrylic cross-linked polymer (Carbomer), and sodium alginate, wherein the ethylene oxide is a water-soluble resin, and the water-soluble resin preferably has a molecular weight of 900,000 Da to 10,000,000 Da, and the specific trade name is polyoxyethylene N80, polyoxyethylene N750, polyoxyethylene 1105, polyoxyethylene N60K, and the like. If various sustained release materials are mixed for use, the mixing ratio does not need to be particularly limited. Meanwhile, the sustained release material of the present invention is present in an amount of 6-60%, preferably 10-50%, and more preferably 15-45% by weight, relative to the weight of the corresponding sustained release component I or the site-specific release component II in the pharmaceutical formulation, and specifically 20%, 25%, 30%, 35%, 40%, or 45% by weight.

In the pharmaceutical formulation of the present invention, the sustained release component I and the site-specific release component II also contain pharmaceutical excipients. Such excipients are well known to the person skilled in the art, and may be one or more of fillers, surfactants, glidants, lubricants, and coatings.

The filler used in the present invention is a water-soluble or water-swellable filler. The water-swellable filler refers to a pharmaceutical excipient that swells after adding water. The water-soluble filler includes dextrin, lactose, sucrose, mannitol, and calcium hydrogen phosphate. The water-swellable filler includes pregelatinized starch, gelatinized starch, microcrystalline (crystalline) cellulose, corn starch, hydroxypropyl methylcellulose (HPMC-K100LV), calcium sulfate, sodium carboxymethyl starch, carboxymethyl cellulose (carboxymethyl cellulose), carmellose calcium, croscarmellose sodium (croscarmellose sodium), soy lecithin, low-substituted hydroxypropyl cellulose, tragacanth powder and bentonite. These water-soluble or water-swellable additives can be used alone or in combination of two or more types, preferably one or more of microcrystalline cellulose, pregelatinized starch, corn starch, dextrin, lactose, sucrose, mannitol, calcium sulfate, and calcium hydrogen phosphate. The filler is preferably present in an amount of 10-40% by weight, which can be 15%, 20%, 25%, 30%, 35%, or 40% by weight, relative to the weight of the corresponding sustained release component I or the site-specific release component II in the pharmaceutical formulation.

In the present invention, "... present in an amount of... relative to the weight of the corresponding sustained release component I or the site-specific release component II in the pharmaceutical formulation" means the weight amount of the pharmaceutical excipients in the corresponding components (sustained release component I or the site-specific release component II). For example, "the filler is present in an amount of 10-40% by weight, relative to the weight of the corresponding sustained release component I or the site-specific release component II in the pharmaceutical formulation" means that filler in the sustained release component I is present in an amount of 10-40% by weight, relative to the weight of the sustained release component I in the pharmaceutical formulation, and the filler in the sustained release component II is present in an amount of 10-40% by weight, relative to the weight of the site-specific release component II in the pharmaceutical formulation.

In the present invention, the expression such as "10-40% by weight of a filler" means that the filler is present in an amount of 10-40% by weight, relative to the weight of the corresponding sustained release component I or the site-specific release component II in the pharmaceutical formulation is 10-40%. Moreover, during the preparation of the pharmaceutical formulation, the person skilled in the art habitually considers the active ingredient, sustained release materials, and pharmaceutical excipients as a whole, and calculates the used coating agent as an additional amount. However, when calculating the range of use or the range of amount of the active ingredient, sustained release materials, or pharmaceutical excipients, the calculation is based on the weight of the entire component, and the present invention is also applicable, see Example 2 for details.

The surfactant according to the present invention includes an ionic surfactant and a nonionic surfactant. The ionic surfactant is stearic acid, sodium lauryl sulfate, lecithin, amino acid and the like. The nonionic surfactant is glyceryl monostearate, polysorbate, sorbitan fatty acid ester, polyoxyethylene-polyoxypropylene copolymer (poloxamer), sodium lauryl sulphonate and the like. The surfactant is preferably present in an amount of 0.5%-10% by weight, which can be 1%, 2%, 3%, 4%, 5%, 6%, 7%, 8%, or 9% by weight, relative to the weight of the corresponding sustained release component I or the site-specific release component II in the pharmaceutical formulation.

The glidant according to the present invention can be hydrated silica (colloidal silica), light anhydrous silicic acid, crystalline cellulose, synthetic aluminum silicate, titanium oxide, stearic acid, calcium stearate, magnesium stearate, tricalcium phosphate, talc, corn starch or aluminum metasilicate, preferably colloidal silica. The glidant is preferably in an amount of 0.1-5% by weight, which can be 0.5%, 1%, 1.5%, 2%, 2.5%, 3%, 3.5%, 4%, or 4.5% by weight, relative to the weight of the corresponding sustained release component I or the site-specific release component II in the pharmaceutical formulation.

The lubricant according to the present invention can be one or more of cocoa butter, carnauba wax, hydrated silica (colloidal silica), aluminum hydroxide xerogel, glycerin fatty acid ester, magnesium silicate, light anhydrous silicic acid, crystalline cellulose, hardened oil, synthetic aluminum silicate, white beeswax, magnesium oxide, sodium potassium tartrate, sucrose fatty acid ester, stearic acid, calcium stearate, magnesium stearate, polyethylene glycol 6000, sodium stearyl fumarate, stearyl alcohol, polyethylene glycol 40 stearate, talc, hydrogenated castor oil, and glyceryl behenate, preferably one or more of stearic acid, magnesium stearate, calcium stearate, polyethylene glycol 6000, sodium stearyl fumarate, talc, hydrogenated castor oil, and glyceryl behenate. The lubricant is preferably in an amount of 0.1-5% by weight, which can be 0.5%, 1%, 1.5%, 2%, 2.5%, 3%, 3.5%, 4%, or4.5% by weight, relative to the weight of the corresponding sustained release component I or the site-specific release component II in the pharmaceutical formulation.

The coating agent (non-site-specific release coating) according to the present invention can be hypromellose, methyl cellulose, ethyl cellulose, methyl cellulose or hydroxypropyl cellulose, polyvinyl alcohol, polyvinylpyrrolidone, polyvinyl acetate resin or polyvinyl acetal diethylamino acetate, aminoalkyl methacrylate copolymer RS and ethyl acrylate-methyl methacrylate copolymer dispersion, sugars including sugar alcohol sucrose, mannitol paste, or commercial product Opadry.

In the pharmaceutical formulation according to the present invention, the sustained release component I comprises (A) 5-20% by weight of apremilast or a pharmacologically acceptable salt or solvate thereof, (B) a sustained release material which is one or two selected from the group consisting polyoxyethylene, hypromellose and hydroxypropyl cellulose; wherein preferably the content of the sustained release material is preferably present in an amount of 6-60%, preferably 10-50%, and more preferably 15-45% by weight, relative to the weight of the corresponding sustained release component I or the site-specific release component II in the pharmaceutical formulation, (C) 10-40% by weight of a filler, (D) 0.5-10% by weight of a surfactant, (E) 0.1-5% by weight of a glidant, (F) 0.1-5% by weight of a lubricant; and the site-specific release component II comprises (A) 5-20% by weight of apremilast or a pharmacologically acceptable salt or solvate thereof, (B) 10-40% by weight of a filler, (C) 0.5-10% by weight of a surfactant, (D) 0.1-5% by weight of a lidant, (E) 0.1- 5% by weight of a lubricant, (F) a site-specific coating material, wherein the site-specific coating material is an enteric coating, which is preferably at least one of ethyl cellulose, hydroxypropyl cellulose, hydroxypropyl methyl cellulose, and acrylic resin; wherein the enteric coating is preferably present in an amount of 1-40%, more preferably 2-30%, and most preferably 2-20% by weight, relative to the weight of the site-specific release component II.

In the pharmaceutical formulation according to the present invention, the weight ratio of the sustained-release component I to the site-specific release component II is 1:8-8:1, preferably 1:6-6:1, and more preferably 1:5-4:1, specifically 2/5, 3/5, 4/5, 1/4, 1/3, 2/3, 3/4, 1/2, 1/1, 2/1, 3/2, or 3/1. Meanwhile, in the pharmaceutical formulation of the present invention, the site-specific release component II can also comprise a sustained release material which is polyoxyethylene, hypromellose or hydroxypropyl cellulose. Preferably, the sustained release material is present in an amount of 6-60%, preferably 10-50%, and more preferably 15-45% by weight, relative to the weight of the corresponding sustained release component I or the site-specific release component II in the pharmaceutical formulation.

The sustained release component I in the pharmaceutical formulation of the present invention is a matrix type sustained release component. Specifically, the active ingredient apremilast is distributed in the sustained release matrix, and the sustained release material distributed in the matrix mainly takes the effect of sustained release. The sustained release component I can or can not contain a coating agent.

When the site-specific release component II of the present invention comprises a sustained release material, the active ingredient is also distributed in the sustained release matrix, and the component II is a matrix-type sustained release component. The site-specific release component II according to the present invention can also comprise other type of coating (non-enteric coating), which plays a role of identification and differentiation in the production of pharmaceutical formulations.

The pharmaceutical formulation of the present invention is a solid formulation which is a tablet, a granule, a powder (including fine granule), a pellet, or a capsule, preferably a capsule or a tablet. The solid formulation can be prepared by widely known preparation methods including wet granulation, dry granulation, or powder direct compression processes.

When the pharmaceutical formulation of the present invention is in a capsule dosage form, the granules, pellets or powders prepared by widely known preparation methods can be encapsulated in the capsule.

The apremilast of the present invention is a solvate (including hydrate) or pharmacologically acceptable salt or solvate (including hydrate) of the salt. The salt includes hydrochloride, sulfate, hydrobromide, citrate, hydriodate, phosphate, nitrate, benzoate, methanesulfonate, benzenesulfonate, 2-hydroxyethanesulfonate, p-toluenesulfonate, acetate, propionate, oxalate, malonate, succinate, glutarate, adipate, tartrate, maleate, fumarate, malate and mandelate.

The apremilast or the pharmacologically acceptable salt or solvate thereof according to the present invention can be synthesized according to the methods disclosed in US Pat. No. 6,962,940 or J. Med. Chem., 2009, 52, 155-1524, and is also commercially available.

The *in vitro* dissolution rate of the pharmaceutical formulation according to the present invention is tested according to the second method, i.e., paddle method of the dissolution test of Chinese Pharmacopoeia, wherein 900 mL of dissolution medium is used, firstly the formulation is placed in a medium with pH 1.0 and tested for 2 hours, and then placed in a phosphate buffer solution with pH 6.8 to determine the dissolution rate, the temperature of the dissolution medium is 37±0.5°C, and the paddle speed is 75 rpm. Samples are taken at 2, 4, 6, 8, 12 hours and measured by a UV spectrophotometer at 230 nm. The dissolution profile is as follows:
10-20% by weight of apremilast is released after 2 hours,
30-60% by weight of apremilast is released after 4 hours,
85-96% by weight of apremilast is released after 8 hours, and
96-100% by weight of apremilast is released after 12 hours.

The numerical values of the amount and the dissolution rate of the invention have unavoidable experimental errors, and the error value is ±1%.

According to the present invention, two apremilast components with different sustained release behaviors are combined according to a certain ratio into a final sustained release formulation, which has a constant dissolution rate in the *in vitro* dissolution test. The *in vitro* dissolution rate and pharmacokinetics of the present sustained release formulation have the following beneficial effects compared with the pharmacokinetics of the ordinary formulations:
1. The plasma concentration can be maintained for a long period of time, thereby avoiding the peak-valley phenomenon of frequent administration of ordinary formulations; meanwhile, drug bioavailability is increased, and the side effects of apremilast on the gastrointestinal tract during drug medication are reduced.
2. The action time is prolonged by different drug release mechanisms, the frenquence of administrations is reduced, and the patients' compliance is improved.

### DESCRIPTION OF THE DRAWINGS

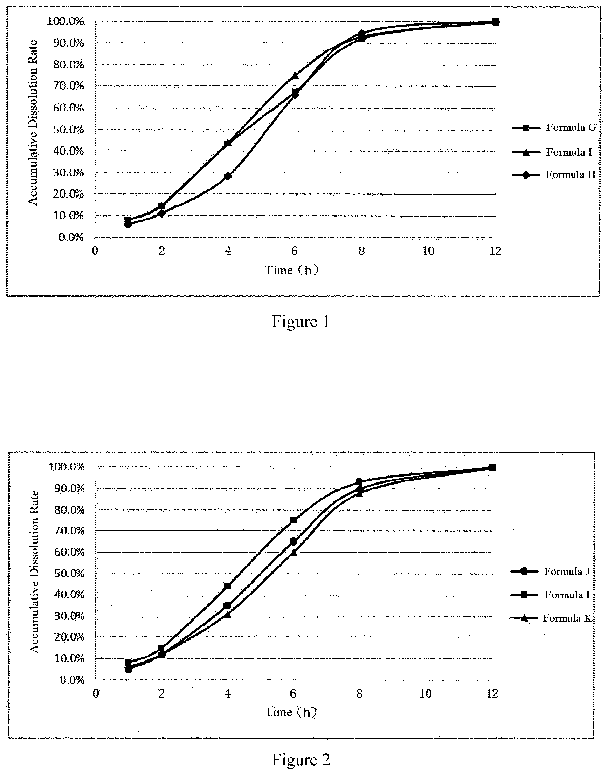

Figure 1 shows the dissolution profile of apremilast in embodiments G, I, and H.
Figure 2 shows the dissolution profile of apremilast in embodiments J, I, and K.

### DETAILED DESCRIPTION OF THE INVENTION

The invention is further illustrated in detail by the following examples. These examples are for illustrative purposes only and are not intended to limit the scope of the invention.

### Example 1

### Preparation of sustained release component I

Apremilast, a filler, sustained release material, surfactant, glidant, and lubricant were mixed according to the ratio in Table 1, and then directly compressed into tablets using a powder direct tabletting technology. Dies with any diameters can be selected when tabletting, including any circular dies with a diameter from 2 mm to 6 mm. The tablet weight was from 10 mg to 100 mg.

**Table 1 (6 mm circular die, each tablet weight of 100 mg)**

| Formula | A | | B | | C | |
|---|---|---|---|---|---|---|
| | Dosage (mg) | Ratio (%) | Dosage (mg) | Ratio (%) | Dosage (mg) | Ratio (%) |
| Apremilast | 15 | 15 | 15 | 15 | 15 | 15 |
| Pregelatinized starch | 10 | 10 | 10 | 10 | 30 | 30 |
| Microcrystalline cellulose | 30 | 30 | 30 | 30 | 30 | 30 |
| HPMC-K100LV | 40 | 40 | / | / | / | / |
| Polyoxyethylene 1105 | / | / | 40 | 40 | 20 | 20 |
| Sodium dodecyl sulfate | 3 | 3 | 3 | 3 | 3 | 3 |
| Silica | 1 | 1 | 1 | 1 | 1 | 1 |
| Magnesium stearate | 1 | 1 | 1 | 1 | 1 | 1 |
| Total Core | 100 | 100 | 100 | 100 | 100 | 100 |

### Example 2

### Preparation of site-specific release component II

Apremilast, a filler, sustained release material, surfactant, glidant, and lubricant were mixed according to the ratio in Table 2, and then directly compressed into tablets using a powder direct tabletting technology. The compressed tablet is then coated with a highly effective coating agent. Dies with any diameters can be selected when tabletting, including any circular dies with a diameter from 2 mm to 6 mm. The tablet weight was from 10 mg to 100 mg.

**Table 2 (6 mm circular die, each tablet weight of 100 mg)**

| Formula | D | | E | | F | |
|---|---|---|---|---|---|---|
| | Dosage (mg) | Ratio (%) | Dosage (mg) | Ratio (%) | Dosage (mg) | Ratio (%) |
| Apremilast | 15 | 15 | 15 | 15 | 15 | 15 |
| Pregelatinized starch | 30 | 10 | 10 | 10 | 30 | 30 |
| Microcrystalline cellulose | 30 | 30 | 30 | 30 | 30 | 30 |
| HPMC-K100LV | 20 | 20 | / | / | / | / |
| Polyoxyethylene 1105 | / | / | 10 | 10 | / | / |
| Polyoxyethylene N60K | / | / | 30 | 30 | 40 | 40 |
| Sodium dodecyl sulfate | 3 | 3 | 3 | 3 | 3 | 3 |
| Silica | 1 | 1 | 1 | 1 | 1 | 1 |
| Magnesium stearate | 1 | 1 | 1 | 1 | 1 | 1 |
| Total core | 100 | 100 | 100 | 100 | 100 | 100 |
| Opadry 85F23718 | 2 | / | 2 | / | 2 | / |
| Eudragit (L100-55) | 10 | / | 10 | / | 10 | / |

### Example 3

### Preparation of apremilast sustained release formulation

The sustained release component I and the site-specific release component II were filed into 00# capsules according to different ratios to provide sustained release formulations with different dissolution profiles. The specific embodiments are shown in Table 3.

**Table 3**

| Embodiment | G | | H | | I | | J | | K | |
|---|---|---|---|---|---|---|---|---|---|---|
| Component | Dosage (mg) | Ratio (%) | Dosage (mg) | Ratio (%) | Dosage (mg) | Ratio (%) | Dosage (mg) | Ratio (%) | Dosage (mg) | Ratio (%) |
| Formula A | 400 | 80 | / | / | / | / | / | / | / | / |
| Formula B | / | / | 400 | 80 | / | / | / | / | / | / |
| Formula C | / | / | / | / | 400 | 80 | 300 | 60 | 300 | 60 |
| Formula D | 100 | 20 | / | / | / | / | / | / | / | / |
| Formula E | / | / | 100 | 20 | / | / | / | / | 200 | 40 |
| Formula F | / | / | / | / | 100 | 20 | 200 | 40 | / | / |

The in vitro dissolution rate of the apremilast sustained release capsules of embodiments G to K were tested according to the second method, i.e., paddle method of the dissolution test of Chinese Pharmacopoeia, wherein 900 mL of dissolution medium is used, firstly the formulation is placed in a medium with pH 1.0 and tested for 2 hours, and then placed in a phosphate buffer solution with pH 6.8 to determine the dissolution rate, the temperature of the dissolution medium is 37±0.5°C, and the paddle speed is 75 rpm. Samples are taken at 2, 4, 6, 8, 12 hours and measured by a UV spectrophotometer at 230 nm. The results are shown in Table 4.

**Table 4 Results of dissolution rate of different embodiments**

| Time (h) | Dissolution Rate (%) | | | | |
|---|---|---|---|---|---|
| | G | H | I | J | K |
| 2 | 14.7 | 11.3 | 15.0 | 12.0 | 12.0 |
| 4 | 43.3 | 28.4 | 44.0 | 35.0 | 31.0 |
| 6 | 67.3 | 66.0 | 75.0 | 65.0 | 60.0 |
| 8 | 92.0 | 94.8 | 93.0 | 90.0 | 88.0 |
| 12 | 99.8 | 99.6 | 100.1 | 99.6 | 99.9 |

From the results shown in the above table, it can be seen that the apremilast sustained release formulation prepared according to the method of the present invention releases slowly within 2 hours, and can achieve the therapeutic effect meanwhile the side effects caused by excessive rapid release after oral administration are avoided. The release is gentle within 2-12 hours, which can ensure a gentle plasma concentration and a lasting effect.

## Claims

1. A pharmaceutical formulation, **characterized in that** it comprises:
I) a sustained release component of apremilast, and
II) a site-specific release component of apremilast.

2. The pharmaceutical formulation according to claim 1, **characterized in that** the sustained release component I comprises:
A) apremilast or a pharmacologically acceptable salt or solvate thereof, preferably in an amount of 5-20% by weight, relative to the weight of the sustained release component I; and
B) a sustained release material.

3. The pharmaceutical formulation according to claim 1 or 2, **characterized in that** the site-specific release component II comprises:
A) apremilast or a pharmacologically acceptable salt or solvate thereof, preferably in an amount of 5-20% by weight, relative to the weight of the sustained release component II; and
B) a site-specific release coating.

4. The pharmaceutical formulation according to claim 3, **characterized in that** the site-specific release coating is an enteric coating or a gastric coating, preferably an enteric coating; wherein the enteric coating is one or more of ethyl cellulose, hydroxypropyl cellulose, hydroxypropyl methyl cellulose, hydroxypropyl methyl cellulose titanate, hydroxypropyl methylcellulose phthalate, hydroxypropyl methylcellulose acetate succinate, polyvinyl acetate phthalate, and acrylic resin; preferably ethyl cellulose, hydroxypropyl cellulose, hydroxypropyl methyl cellulose, or acrylic resin; wherein the site-specific release coating is preferably present in an amount of 1-40%, more preferably 2-30%, and most preferably 2-20% by weight, relative to the weight of the site-specific release component II.

5. The pharmaceutical formulation according to claim 2, **characterized in that** the sustained release material in the sustained release component I is one or more of hypromellose, polyoxyethylene, hydroxypropyl cellulose, copolymer of polyalkyl saccharose or polyalkyl pentaerythritol and acrylic cross-linked polymer, and sodium alginate.

6. The pharmaceutical formulation according to claim 3, **characterized in that** the site-specific release component II also comprises a sustained release material which is one or more of hypromellose, polyoxyethylene, hydroxypropyl cellulose, copolymer of polyalkyl saccharose or polyalkyl pentaerythritol and acrylic cross-linked polymer, and sodium alginate.

7. The pharmaceutical formulation according to claim 5 or 6, **characterized in that** the sustained release material is present in an amount of 6-60%, preferably 10-50%, and more preferably 15-45% by weight, relative to the weight of the corresponding sustained release component I or the site-specific release component II in the pharmaceutical formulation.

8. The pharmaceutical formulation according to any one of claims 1 to 7, **characterized in that** the weight ratio of the sustained release component I to the site-specific release component II is 1:8-8:1, preferably 1:6-6:1, and more preferably 1:5-4:1.

9. The pharmaceutical formulation according to claim 2, **characterized in that** the sustained release component I also comrpsies a pharmaceutical excipient which is one or more selected from the group consisting of a filler, surfactant, glidant, lubricant, and coating agent.

10. The pharmaceutical formulation according to claim 3, **characterized in that** the site-specific release component II also comprises a pharmaceutical excipient which is one or more selected from the group consisting of a filler, surfactant, glidant, lubricant, and coating agent.

11. The pharmaceutical formulation according claim 9 or 10, **characterized in that** the filler is a water-soluble filler or a water-swellable filler, and is preferably one or more selected from the group consisting of microcrystalline cellulose, pregelatinized starch, corn starch, dextrin, lactose, sucrose, mannitol, calcium sulfate, and calcium hydrogen phosphate; the filler is preferably present in an amount of 10-40% by weight, relative to the weight of the corresponding sustained release component I or the site-specific release component II in the pharmaceutical formulation.

12. The pharmaceutical formulation according claim 9 or 10, **characterized in that** the surfactant is one or more of ionic surfactant or nonionic surfactant; the ionic surfactant is stearic acid, sodium lauryl sulfate, lecithin, or amino acid, and the nonionic surfactant is glyceryl monostearate, polysorbate, sorbitan fatty acid ester, polyoxyethylene-polyoxypropylene copolymer; the surfactant is preferably present in an amount of 0.5%-10% by weight, relative to the weight of the corresponding sustained release component I or the site-specific release component II in the pharmaceutical formulation.

13. The pharmaceutical formulation according claim 9 or 10, **characterized in that** the glidant is one or more of silica, talc, and micro powder silica gel; the glidant is preferably present in an amount of 0.1-5% by weight, relative to the weight of the corresponding sustained release component I or the site-specific release component II in the pharmaceutical formulation.

14. The pharmaceutical formulation according claim 9 or 10, **characterized in that** the lubricant is one or more selected from the group consisting of stearic acid, magnesium stearate, calcium stearate, polyethylene glycol 6000, sodium stearyl fumarate, talc, hydrogenated castor oil, and glyceryl behenate; the lubricant is preferably present in an amount of 0.1-5% by weight, relative to the weight of the corresponding sustained release component I or the site-specific release component II in the pharmaceutical formulation.

15. The pharmaceutical formulation according any one of claims 1 to 14, **characterized in that** the pharmaceutical formulation is a granule, powder, tablet, capsule, suspension, or pill.

16. The pharmaceutical formulation according to claim 1, wherein the sustained release component I comprises:
A) 5-20% by weight of apremilast or a pharmacologically acceptable salt or solvate thereof;
B) a sustained release material which is one or two selected from the group consisting of polyoxyethylene, hypromellose and hydroxypropyl cellulose; wherein the sustained release material is preferably present in an amount of 6-60%, preferably 10-50%, and more preferably 15-45% by weight, relative to the weight of the corresponding sustained release component I or the site-specific release component II in the pharmaceutical formulation,
C) 10-40% by weight of a filler;
D) 0.5-10% by weight of a surfactant;
E) 0.1-5% by weight of a glidant; and
F) 0.1-5% by weight of a lubricant.

17. The pharmaceutical formulation according to claim 1, **characterized in that** the site-specific release component II comprises:
A) 5-20% by weight of apremilast or a pharmacologically acceptable salt or solvate thereof;
B) 10-40% by weight of a filler;
C) 0.5-10% by weight of a surfactant;
D) 0.1-5% by weight of a glidant;
E) 0.1-5% by weight of a lubricant; and
F) an enteric coating which is preferably at least one of ethyl cellulose, hydroxypropyl cellulose, hydroxypropyl methyl cellulose, and acrylic resin; wherein the enteric coating is preferably present in an amount of 1-40%, more preferably 2-30%, and most preferably 2-20% by weight, relative to the weight of the site-specific release component II.

18. The pharmaceutical formulation according to claim 17, **characterized in that** the site-specific release component II also comprises a sustained release material which is polyoxyethylene, hypromellose or hydroxypropyl cellulose; the sustained release material is preferably present in an amount of 6-60%, preferably 10-50%, and more preferably 15-45% by weight, relative to the weight of the corresponding site-specific release component II in the pharmaceutical formulation.

19. The pharmacetucial formulation according to any one of claims 16 to 18, **characterized in that** the weight ratio of the sustained release component I to the site-specific release component II is 1:8-8:1, preferably 1:6-6:1, and more preferably 1:5-4:1.

20. A sustained release formulation comprising apremilast, **characterized in that** the following dissolution profile is obtained:
10-20% by weight of apremilast is released after 2 hours,
30-60% by weight of apremilast is released after 4 hours,
85-96% by weight of apremilast is released after 8 hours, and
96-100% by weight of apremilast is released after 12 hours;
when tested according to the second method, i.e., paddle method of the dissolution test of Chinese Pharmacopoeia, wherein 900 mL of dissolution medium is used, firstly the formulation is placed in a medium with pH 1.0 and tested for 2 hours, and then placed in a phosphate buffer solution with pH 6.8, the temperature of the dissolution medium is 37±0.5°C, the paddle speed is 75 rpm, and a UV spectrophotometer is used at 230 nm.

21. A pharmaceutical formulation according to any one of claims 1 to 19, having the dissolution profile according to claim 20.
